# EUROPEAN PATENT SPECIFICATION

(11) **EP 3 418 927 B1**
(45) Date of publication and mention of the grant of the patent: **01.10.2025**
(21) Application number: 17798745.0
(22) Date of filing: 17.05.2017
(51) Int. Cl.: G16B 30/20, G16B 30/10

(54) **METHOD AND DEVICE FOR PROCESSING DNA SEQUENCE**
VERFAHREN UND VORRICHTUNG ZUR VERARBEITUNG EINER DNA-SEQUENZ
PROCÉDÉ ET DISPOSITIF POUR TRAITER UNE SÉQUENCE D'ADN

(30) Priority: 18.05.2016 CN 201610330331
(43) Date of publication of application: 26.12.2018
(73) Proprietor: Huawei Technologies Co., Ltd., Longgang District Shenzhen, Guangdong 518129 (CN)
(72) Inventor: DENG, Liqun, Shenzhen Guangdong 518129 (CN); WEI, Jiansheng, Shenzhen Guangdong 518129 (CN); ZHANG, Jun, Shenzhen Guangdong 518129 (CN)
(74) Representative: Epping - Hermann - Fischer
(86) International application number: PCT/CN2017/084757
(87) International publication number: WO 2017/198182

(56) References cited:
- CN-A- 103 725 773
- CN-A- 103 820 313
- CN-A- 104 087 505
- CN-A- 104 239 732
- DRIES DECAP ET AL: "Halvade: scalable sequence analysis with MapReduce", BIOINFORMATICS., vol. 31, no. 15, 1 August 2015 (2015-08-01), GB, pages 2482 - 2488, XP055565677, ISSN: 1367-4803, DOI: 10.1093/bioinformatics/btv179
- ROBERT BUKOWSKI ET AL: "Variant calling: Part 1", 27 June 2015 (2015-06-27), XP055566329, Retrieved from the Internet <URL:https://biohpc.cornell.edu/lab/doc/Variant_workshop_Part1.pdf> [retrieved on 20190308]
- KRISTOPHERA STANDISH ET AL: "Group-based variant calling leveraging next-generation supercomputing for large-scale whole-genome sequencing studies", BMC BIOINFORMATICS, BIOMED CENTRAL LTD, LONDON, UK, vol. 16, no. 1, 22 September 2015 (2015-09-22), pages 1 - 14, XP021237342, DOI: 10.1186/S12859-015-0736-4
- CUMMINGS NIK ET AL: "Combining target enrichment with barcode multiplexing for high throughput SNP discovery", BMC GENOMICS, BIOMED CENTRAL LTD, LONDON, UK, vol. 11, no. 1, 18 November 2010 (2010-11-18), pages 641, XP021086242, ISSN: 1471-2164, DOI: 10.1186/1471-2164-11-641
- PUCKELWARTZ MEGAN J ET AL: "Supercomputing for the parallelization of whole genome analysis", BIOINFORMATICS (OXFORD, ENGLAND), 12 February 2014 (2014-02-12), England, pages 1508 - 1513, XP055831484, Retrieved from the Internet <URL:https://watermark.silverchair.com/btu071.pdf?token=AQECAHi208BE49Ooan9kkhW_Ercy7Dm3ZL_9Cf3qfKAc485ysgAAAvYwggLyBgkqhkiG9w0BBwagggLjMIIC3wIBADCCAtgGCSqGSIb3DQEHATAeBglghkgBZQMEAS4wEQQMMST_Y2w1vd028SmXAgEQgIICqTEc-6uYLmB2jT90bmmeeWc5yHootnIDoLMi3IY2ZMQgYocFTfYZb7bZa9eZXwvEQDd1N0tLh37vjykbXnaL8BFDgssh6> [retrieved on 20210811], DOI: 10.1093/bioinformatics/btu071

## Description

### TECHNICAL FIELD

The present invention relates to the field of genetic engineering, and in particular, to a DNA sequence processing method and device.

### BACKGROUND

A gene is a function fragment that is of a deoxyribonucleic acid (Deoxyribonucleic acid, DNA) molecule and that carries genetic information. The gene supports a basic construction and performance of a living thing. Not all DNA molecules are genes. In the prior art, there is a mature processing procedure for a DNA sample. The procedure generally includes three steps: DNA sequencing, DNA sequence determining, and gene positioning and mutation detection. DNA sequencing indicates that a DNA sequencer is used to extract DNA of a biological sample and convert the extracted DNA into a data sequence read that can be identified by a computer. Specifically, a base sequence in a DNA sequence is identified by using a chemical method. The base sequence includes four linked bases: A, C, T, and G. Then, the base sequence is converted into a character string sequence that can be identified by a computer and that includes four characters: A, C, T, and G. One read is a DNA fragment with a fixed length, and is a basic unit for subsequent DNA sequence processing. For example, if a length of one read is 10 base pairs (Base Pair, BP), a read data sequence ATCTGTCCTA is one read.

A result of DNA sequencing is that a large quantity of DNA reads are generated, but an order of these reads is unknown. A function of DNA sequence determining is to map these disordered DNA reads to a recognized DNA reference sequence (that is, alignment computation) one by one, to obtain an optimal match location of each read in the reference sequence.

Gene positioning and mutation detection is to determine genes in the DNA sequence by using gene information pre-saved in a database, and compute a mutation site and a mutation degree of the genes relative to a gene template in the database.

In the prior art, multiple different sequencers exist, and different sequencers have different sequencing libraries. The sequencing library may be understood as a kind of chemical culture reagent, to help a sequencer extract and identify DNA from a biological sample. Different chemistry culture reagents have different identification precision for bases in the DNA. In the existing DNA sequence processing procedure from DNA sequence determining to gene mutation detection, only a read group of one sequencing library is processed each time, and this is inefficient.

DRIES DECAP ET AL: "Halvade: scalable sequence analysis with MapReduce" presents the Halvade framework that enables sequencing pipelines to be executed in parallel on a multi-node and/or multi-core compute infrastructure in a highly efficient manner. For example, a DNA sequencing analysis pipeline for variant calling has been implemented according to the GATK Best Practices recommendations, supporting both whole genome and whole exome sequencing.

PUCKELWARTZ ET AL: "Supercomputing for the parallelization of whole genome analysis" presents the MegaSeq workflow to use the size and memory of the Cray XE6 super computer for whole genome analysis. The Cray XE6 is adapted to improve the parallelization for concurrent multiple genome analysis.

### SUMMARY

The invention is set out in the appended set of claims. An objective of the present invention is to provide a DNA sequence processing method and device, to resolve a prior-art problem of low-efficiency gene mutation detection on a DNA sample.

To achieve the foregoing objective, the present invention uses the following technical solutions:
According to a first aspect, a DNA sequence processing method is provided, and the method is used to process N read groups of a deoxyribonucleic acid DNA sample. Each read group includes sequence fragments reads that are obtained after a corresponding sequencing library is used to perform sequencing on the DNA sample, and N is a positive integer greater than 1. The method includes: performing the following operations on each read group concurrently: performing alignment computation on each read in the read group according to a reference sequence of a chromosome, to obtain an alignment result record of the read relative to the reference sequence; determining, according to the alignment result record, a chromosome region in which each read is located, where the chromosome includes at least one chromosome region; and merging, into one intermediate result file, alignment result records of reads located in a same chromosome region, where alignment computation is performed on each read group according to a same reference sequence of a chromosome, chromosome regions included in the chromosome are the same, and after the foregoing operations are performed on each read group, each chromosome region is corresponding to N intermediate result files; further, determining a target sequence file of each chromosome region according to the N intermediate result files
corresponding to the chromosome region; and performing mutation detection on the target sequence file of each chromosome region, to determine mutation site information of the chromosome region.

According to the DNA sequence processing method provided in the first aspect, the alignment computation operation is performed on a read group of each sequencing library concurrently. Therefore, a time for processing the DNA sample is shortened, and DNA sequence processing efficiency is improved. In addition, a gene mutation status of the DNA sample is detected according to results that are obtained after multiple sequencing libraries are used to perform sequencing on the DNA sample. Therefore, in comparison with a single sequencing library, detection precision is improved in the DNA sequence processing method provided in the present invention.

With reference to the first aspect, in a first possible implementation of the first aspect, before the performing the operations on each read group concurrently, the method further includes: individually saving each read group into a distributed storage system. Individual storage helps distinguish between different read groups, and helps save an intermediate file generated in a processing process.

With reference to the first possible implementation of the first aspect, in a second possible implementation of the first aspect, the individually saving each read group into a distributed storage system includes: dividing each read group into at least one data block for saving; and the performing alignment computation on each read in the read group according to a reference sequence of a chromosome includes: performing, concurrently according to the reference sequence, alignment computation on a data block corresponding to each read. In this way, data blocks in a read group and read groups of sequencing libraries are processed concurrently, so that processing efficiency is improved.

With reference to any one of the first aspect or the possible implementations of the first aspect, in a third possible implementation of the first aspect, the alignment result record includes an identifier of a chromosome on which each read is located and location information of the read on the chromosome, and the determining, according to the alignment result record, a chromosome region in which each read is located includes: determining, according to the identifier of the chromosome and the location information, the chromosome region in which each read is located on the chromosome. In the third possible implementation of the first aspect, optionally, the reference sequence includes all sequences of the chromosome, and a DNA sequence fragment read is aligned with the reference sequence, to determine a region that is in the reference sequence and that is most similar to the read, so as to determine location information of the read on the chromosome.

With reference to the third possible implementation of the first aspect, in a fourth possible implementation of the first aspect, the merging, into one intermediate result file, alignment result records of reads located in a same chromosome region includes: performing operations that include at least sorting and de-duplicating on the alignment result records of the reads located in the same chromosome region, to obtain the intermediate result file. Optionally, in the fourth possible implementation of the first aspect, a Picard tool may be used to perform regulation operations such as sorting and de-duplicating on the alignment result records, to obtain the intermediate result file. The intermediate result file indicates a data sequence that is of a chromosome region and that is determined after DNA sequence determining is performed on a read group that is obtained by means of sequencing by using a single sequencing library.

According to a second aspect, a computing device is provided, and the computing device is configured to perform the method in any one of the first aspect or the possible implementations of the first aspect. In an implementation of the computing device, the computing device includes units that are configured to perform the method in any one of the first aspect or the possible implementations of the first aspect. In another implementation of the computing device, the computing device includes a processor, a memory, a communication port, and a communications bus. The processor, the memory, and the communication port communicate with each other by using the communications bus. The processor is configured to perform the method in any one of the first aspect or the possible implementations of the first aspect.

According to a third aspect, a computer readable medium is provided, and configured to save a computer program, where the computer program includes instructions that are used to perform the method in any one of the first aspect or the possible implementations of the first aspect.

### BRIEF DESCRIPTION OF DRAWINGS

FIG. 1 is a schematic flowchart of a DNA sequence processing method according to an embodiment of the present invention;
FIG. 2 is a schematic diagram of a storage structure of a read group according to an embodiment of the present invention;
FIG. 3 is a schematic diagram of a storage structure of intermediate sequence information according to an embodiment of the present invention;
FIG. 4A, FIG. 4B, and FIG. 4C are a schematic flowchart of another DNA sequence processing method according to an embodiment of the present invention;
FIG. 5A and FIG. 5B are a schematic flowchart of still another DNA sequence processing method according to an embodiment of the present invention;
FIG. 6 is a schematic structural diagram of a computing device according to an embodiment of the present invention; and
FIG. 7 is a schematic structural diagram of another computing device according to an embodiment of the present invention.

### DESCRIPTION OF EMBODIMENTS

To make it easier for persons skilled in the art to understand improvements in the prior art that are described in the embodiments of the present invention, the following first briefly introduces the solution in the prior art.

A BWA-Picard-GATK based processing procedure is the current industry-recognized optimal practice for implementing DNA sequence determining and mutation detection. The Burrows-Wheeler aligner (Burrows-Wheeler Aligner, BWA) is responsible for performing alignment computation on each data sequence read of DNA sample according to a reference sequence. The Picard tool is responsible for steps such as sorting, de-duplicating, and format conversion of alignment result records. The genome analysis toolkit (The Genome Analysis Toolkit, GATK) is responsible for mutation detection, including three steps: local realignment, base quality recalibration, and mutation identification. In specific operations, these sub-steps are performed in sequence according to command lines submitted by a user.

Halvade is an implementation of the BWA-Picard-GATK procedure in a Hadoop MapReduce system. Halvade makes full use of MapReduce framework features, and integrates steps of DNA sequence processing into corresponding steps of MapReduce. Specific implementation is as follows:
In terms of preprocessing and uploading, a to-be-processed DNA read is divided into individual data blocks with a specific size, so that each data block can be fully written into a single HDFS block after the data is uploaded to an HDFS.

In terms of a map stage, a quantity of map tasks depends on a quantity of HDFS blocks, that is, each HDFS block is corresponding to input of one map task. However, computation content of each map task is to invoke the BWA to perform a mapping operation on DNA reads for which the BWA is responsible, and output a mapping result, that is, <chromosome region, SAM record>. The sequence alignment map (Sequence Alignment Map, SAM) record is a result format that is obtained after a DNA read is mapped to a reference DNA sequence, and the read and a result of aligning the read with the chromosome region are recorded in the sequence alignment map record.

In terms of an intermediate data distribution stage, after the map stage is completed, the MapReduce system sorts intermediate results according to key values of the intermediate results, and distributes the intermediate results to each node in a cluster for processing at a reduce stage.

In terms of the reduce stage, a quantity of reduce tasks is equal to a quantity of chromosome regions, that is, one reduce task is initiated for each chromosome region, and the tasks are performed concurrently in the cluster. In each reduce task, the Picard and the GATK are run in sequence to complete corresponding computation steps. For each reduce task, one mutation site detection result file is finally generated for the chromosome region, and the detection result file is saved in a vcf file format.

In terms of result merging, all vcf files generated at the reduce stage are merged into a unified vcf file, and the unified vcf file is used as output of the entire procedure.

However, currently each time Halvade can process data generated by only one sequencing library. When multiple sequencing libraries exist, the existing Halavde can perform only the foregoing procedure once for data generated by each library, and this is inefficient.

To make the objectives, technical solutions, and advantages of the embodiments of the present invention clearer, the following clearly and completely describes the technical solutions in the embodiments of the present invention with reference to accompanying drawings in the embodiments of the present invention. Apparently, the described embodiments are some but not all of the embodiments of the present invention. All other embodiments obtained by persons of ordinary skill in the art based on the embodiments of the present invention without creative efforts shall fall within the protection scope of the present invention.

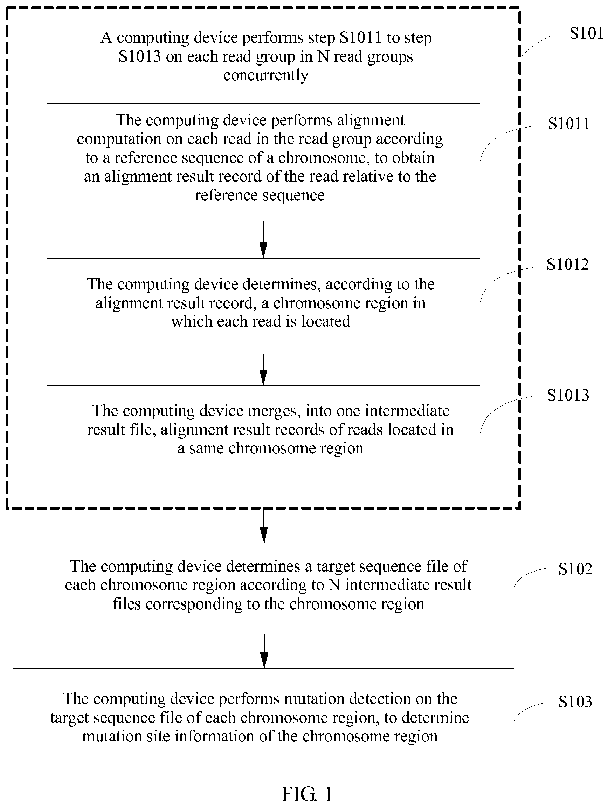

An embodiment of the present invention provides a DNA sequence processing method. The method is used to process N read groups of a DNA sample, each read group includes sequence fragments reads that are obtained after a corresponding sequencing library is used to perform sequencing on the DNA sample, and N is a positive integer greater than 1. That is, N sequencing libraries are used to separately perform sequencing on a same DNA sample, and a group of multiple sequence fragments obtained after each sequencing library is used to perform sequencing is a read group. As shown in FIG. 1, the method includes the following steps.

S101. A computing device performs step S1011 to step S1013 on each read group in the N read groups concurrently.

It should be noted that one sequencing library may include multiple corridors, and each corridor has a different configuration and focuses on a different aspect of DNA sequencing. The read group includes data sequences reads of all corridors in one library. The present invention sets no limitation to construction of the sequencing library.

S1011. The computing device performs alignment computation on each read in the read group according to a reference sequence of a chromosome, to obtain an alignment result record of the read relative to the reference sequence.

The reference sequence may be all data sequences of the chromosome used for reference. In specific implementation, the reference sequence may be a current industry-recognized DNA reference sequence, and the reference sequence may be pre-saved in the computing device. A format of the alignment result record may be the same as a record format in the prior art, for example, a SAM record.

In this specification, alignment computation performed on the read according to the reference sequence is also referred to as mapping. In specific implementation, step S1011 may be implemented by the computing device by using a BWA.

S1012. The computing device determines, according to the alignment result record, a chromosome region in which each read is located.

The chromosome includes at least one chromosome region.

Specifically, the computing device determines corresponding data division for each alignment result record in a preset alignment data division manner. For example, the alignment result includes an identifier of a chromosome on which a read is located and location information such as start coordinates of the read on the chromosome. In this way, the computing device can determine, in the preset alignment data division manner, an intermediate result data set represented by a chromosome region in which each alignment result record is located.

It should be noted that, according to the alignment data division method, the reference chromosome may be divided into multiple chromosome regions with different lengths (or with a same length). In this way, because the reference sequence is all the data sequences of the reference chromosome, a chromosome region corresponding to each alignment result record may be determined according to a location (jointly determined by an identifier of the chromosome and start coordinates) of the record on a chromosome.

In addition, when determining data division for each alignment result record, the computing device may further convert a format of the alignment result record to an intermediate result record format required by the computing device. The intermediate result record format may be a key-value pair. A combination of an identifier of a chromosome and start coordinates is used as a key (key), and a SAM record is used as a value (value).

S1013. The computing device merges, into one intermediate result file, alignment result records of reads located in a same chromosome region.

Specifically, the computing device performs a mapping result record regulation operation on an intermediate result data set represented by each chromosome region, to obtain the intermediate result file. In this specification, a process that includes sorting (Sort) and de-duplicating (De-Duplicates) operations that are performed on the alignment result data and various preparation operations required before mutation detection is referred to as mapping result record regulation. In specific implementation, the sorting and de-duplicating operations may be completed by the computing device by using a Picard tool. The various preparation operations required before mutation detection may include generating a BAM file. The operations may also be completed by the computing device by using a SAMtools tool.

It should be noted that, the intermediate result data set represented by each chromosome region may be distributed on different computing nodes. In this case, before the mapping result record regulation operation is performed on the intermediate result data set represented by the chromosome region, data at different locations needs to be collected as one temporary intermediate result file (the file may be a SAM file or a BAM file). The foregoing sorting operation may be implemented in a collection process by means of merge sort.

After the mapping result record regulation operation is performed on the intermediate result data set represented by each chromosome region, one SAM file or one BAM file is obtained. The file is the intermediate result file.

It should be noted that, alignment computation is performed on each read group according to a same reference sequence of a chromosome, chromosome regions included in the chromosome are the same, and after the foregoing operations are performed on each read group, each chromosome region is corresponding to N intermediate result files. That is, after the computing device performs step S1011 to step S1013 on a read group of each sequencing library, one intermediate result file of each chromosome region is obtained. Therefore, after the computing device completes the foregoing parallel operations, N intermediate result files corresponding to each chromosome region may be obtained.

S102. The computing device determines a target sequence file of each chromosome region according to N intermediate result files corresponding to the chromosome region.

Specifically, the N intermediate result files corresponding to each chromosome region are merged to generate the target sequence file. It should be noted that, an operation of merging the N intermediate result files may be implemented by using a MergeSamFiles tool of the Picard. In a merging process, the sorting and de-duplicating operations may be performed simultaneously. A merging result is the target sequence file. The file may be a SAM file or a BAM file.

S103. The computing device performs mutation detection on the target sequence file of each chromosome region, to determine mutation site information of the chromosome region.

Specifically, the computing device performs mutation detection on the target sequence file of each chromosome region according to a pre-saved gene database and/or a mutation site database, to obtain a mutation detection result of the target sequence file of the chromosome region.

In specific implementation, the computing device may invoke a GATK tool to implement step S103.

By using the foregoing method, the computing device may detect a gene mutation status of the DNA sample according to results that are obtained after the multiple sequencing libraries are used to perform sequencing on the DNA sample. Because the computing device may perform an alignment computation operation on the read group of each sequencing library concurrently, a time for processing the DNA sample is shortened, and DNA sequence processing efficiency is improved.

To make persons skilled in the art better understand the technical solutions provided in the present invention, the following describes in detail the steps in the foregoing method.

In a possible implementation of this embodiment of the present invention, after obtaining the N read groups that are obtained after the N libraries are used to perform sequencing, the computing device may individually save each read group into a distributed storage system. Specifically, each read group may be individually saved into the distributed storage system in a single file form.

The distributed storage system may be a distributed storage system of the computing device, or may be a distributed storage system of another device connected to the computing device.

In addition, the distributed storage system may be a distributed storage system HDFS provided by a Hadoop platform or may be another distributed storage system. The present invention sets not limitation thereto.

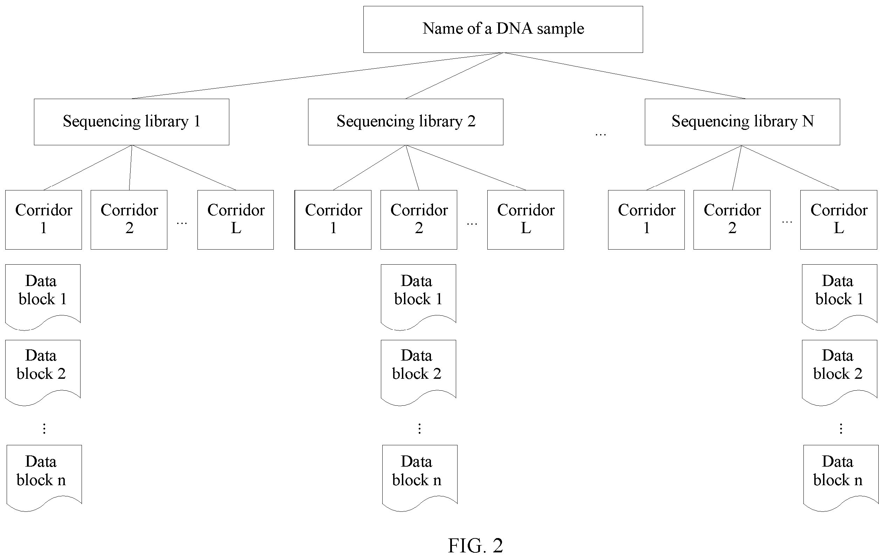

Distributed storage helps the computing device distinguish the read group of each sequencing library. For example, the computing device may obtain a name of the DNA sample, a name of each sequencing library, and a name of each corridor included in each sequencing library. In this way, the computing device may save the read groups according to a directory structure shown in FIG. 2. As shown in FIG. 2, a folder named after the name of the DNA sample includes N folders respectively named after names of the N sequencing libraries, each folder in the N folders includes L folders named after names of corridors in a sequencing library corresponding to the folder, and the L folders save data blocks of reads that are obtained after sequencing is performed by using the corridors. A size of each data block may be set according to an actual requirement. For example, in the HDFS, a size of a data block is 64 megabytes (MB).

It can be learned from the foregoing example that the computing device may divide each read group into at least one data block for saving. In this way, when the computing device performs alignment computation on each read in the read group according to the reference sequence, the computing device may perform, concurrently according to the reference sequence, alignment computation on a data block corresponding to each read.

It should be noted that the data block may be a data block in a Fastq format. Fastq is a text format in which a biometric sequence and a corresponding quality evaluation are saved. In this way, when a mapping operation is performed on a same read, alignment computation may be performed concurrently on all Fastq data blocks of the read according to the reference sequence. The foregoing description is merely an example, and a quantity of corridors included in each sequencing library is not limited in the present invention. Each corridor saves data blocks of a read that is obtained after sequencing is performed by using the corridor, but not all data blocks are shown in FIG. 2.

Optionally, the alignment result includes an identifier of a chromosome on which each read is located and location information of the read on the chromosome. In this case, the computing device may determine, according to the identifier of the chromosome and the location information, the chromosome region in which each read is located.

Optionally, the alignment result further includes similarity information. When the computing device determines an intermediate result file of a chromosome region, if multiple reads are corresponding to a same chromosome identifier and location information of the multiple reads on the chromosome are the same, the computing device selects reads with high similarity in the de-duplicating operation according to the similarity information, to form the intermediate result file of the chromosome region.

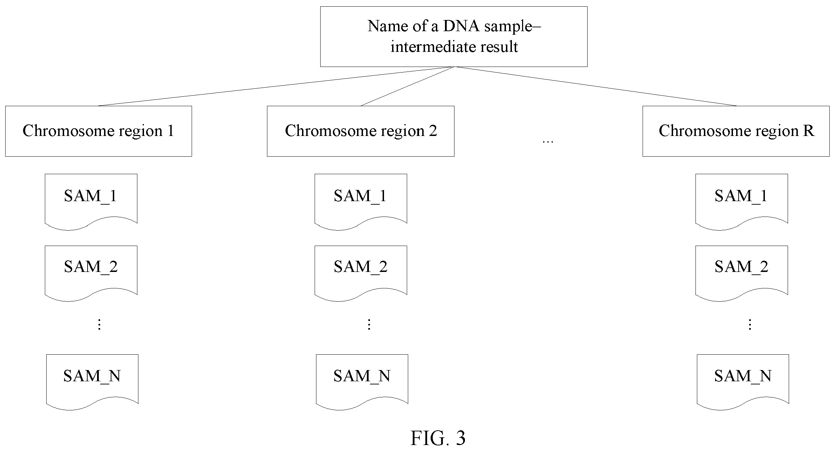

For example, the computing device runs the Picard software, where a data flow that includes alignment result data sets corresponding to a same chromosome region is used as input, and a format of the alignment result record may be a SAM or a BAM; and sequentially runs AddOrReplaceReadGroups and MarkDuplicates commands to complete operations of adding ReadGroup information and removing a duplicate record. Finally, a SAM or BAM file, that is, the intermediate result file, generated by running MarkDuplicates is obtained. Further, the intermediate result file may be uploaded to the distributed storage system for saving. A storage organization form of the intermediate result file may be shown in FIG. 3, a quantity of chromosome regions is R, a two-level file directory form is used, one folder is established for each chromosome region, and SAM files corresponding to the chromosome region are saved in the folder.

Further, the computing device runs a Picard MergeSamFiles command, to merge intermediate sequence information of the chromosome region into a single target sequence file. The target sequence information is recorded in a BAM format, and the BAM is a binary-representation format of the SAM. Then, the computing device runs a Picard BuildBamIndex command to establish an index for the target sequence file, and sequentially runs RealignerTargetCreator, IndelRealigner, BaseRecalibrator, and HaplotypeCaller commands of the GATK, to respectively perform sub-steps such as local realignment, base quality recalibration, and mutation identification, so as to generate a VCF file of a mutation detection result of the chromosome region.

Further, the computing device may further generate a mutation detection result of the DNA sample according to mutation site information of each chromosome region. Specifically, the computing device merges VCF files of the chromosome regions into a single VCF file for output.

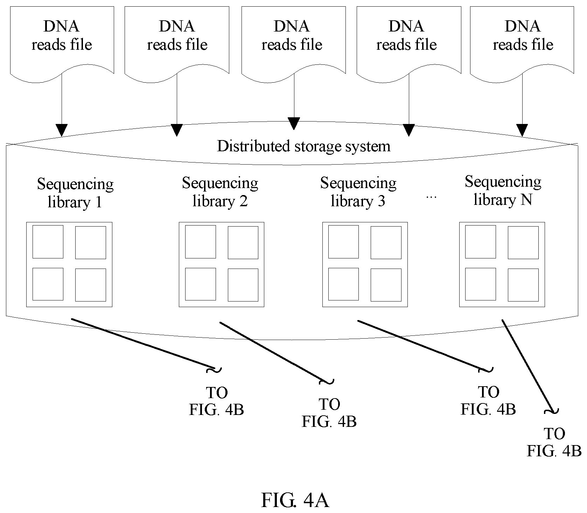

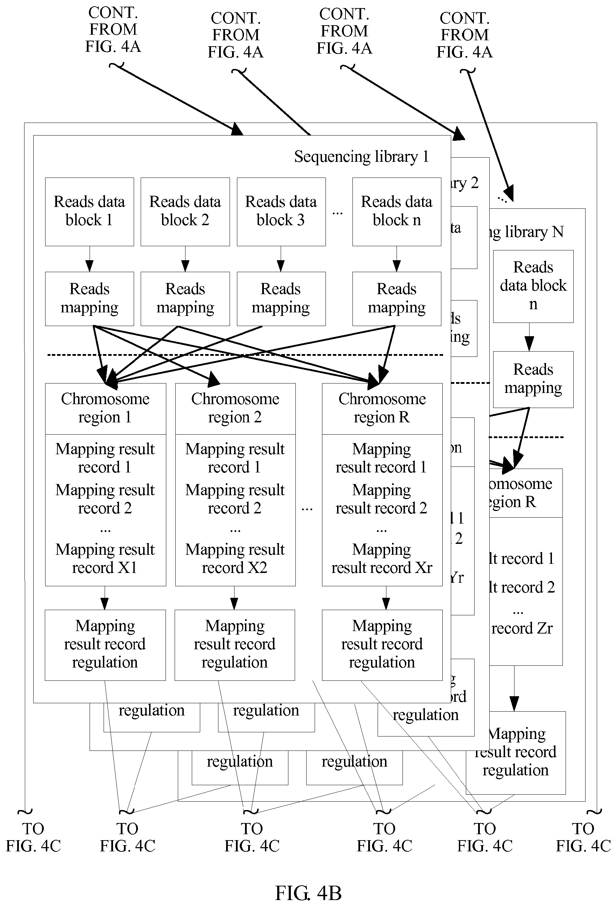

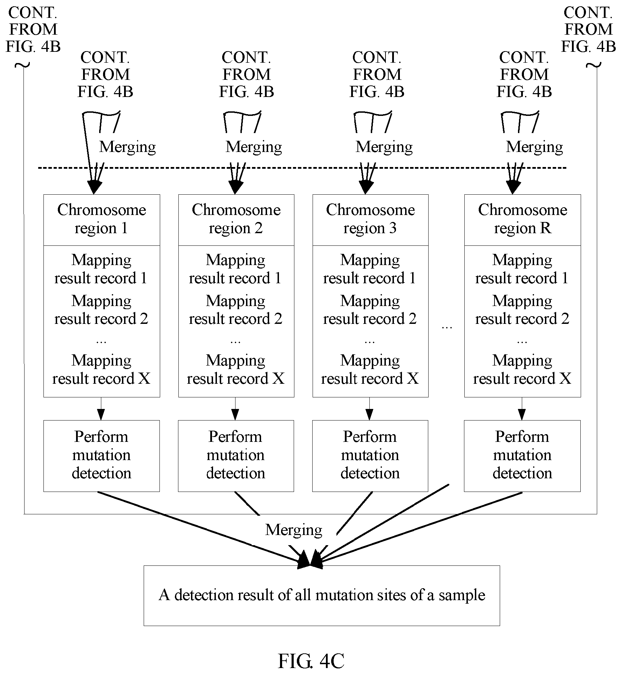

FIG. 4A, FIG. 4B, and FIG. 4C are a schematic flowchart of a DNA sequence processing method according to an embodiment of the present invention. FIG. 4A, FIG. 4B, and FIG. 4C show in detail steps performed by a computing device after the computing device obtains N read groups of N sequencing libraries. Each DNA reads file shown in FIG. 4A, FIG. 4B, and FIG. 4C includes one read group that is obtained after a sequencing library is used to perform sequencing on the DNA sample. In a distributed storage system shown in FIG. 4A, FIG. 4B, and FIG. 4C, the read group of each sequencing library is individually saved, and each read in the read group is divided into data blocks for saving.

In this way, the computing device performs mapping and mapping result regulation in a computation manner of mixing a coarse-grained task and a fine-grained task, that is, mapping or mapping result regulation is performed concurrently on data blocks in a coarse-grained library and data blocks in a fine-grained library. As shown in FIG. 4A, FIG. 4B, and FIG. 4C, the computing device initiates concurrently a running instance for the read group corresponding to each sequencing library, to perform operations such as mapping and mapping result record regulation. In addition, in a process of processing the read group of each sequencing library, the computing device performs mapping operation processing concurrently in a unit of a single data block of a read. As shown in FIG. 4A, FIG. 4B, and FIG. 4C, each read group includes n data blocks. When the mapping operation is performed, the mapping operation is performed concurrently on each data block in a same read group.

After the mapping operation is completed, a generated mapping result record is classified into a corresponding intermediate result set according to a chromosome region to which the mapping result record is mapped relative to a reference sequence. As shown in FIG. 4A, FIG. 4B, and FIG. 4C, for R chromosome regions, each chromosome region is corresponding to X mapping result records. The subsequent result record regulation operation is also performed concurrently in a unit of a chromosome region. After regulation is performed on the X mapping result records, the intermediate result files in step S1013 are obtained.

Further, after the mapping and result record regulation operations are completed, the computing device undergoes a synchronization waiting process, that is, the computing device waits for processing of data of all libraries to be completed, and then goes to a next step. For each chromosome region, one intermediate result file is generated during data processing of each sequencing library. Therefore, in FIG. 4A, FIG. 4B, and FIG. 4C, a merging operation in which result records are regulated to a chromosome region is an operation in which the computing device merges N intermediate result files into one file (that is, the target sequence file).

Further, the computing device performs concurrently a mutation detection operation on each chromosome region according to R target sequence files, and separately generates a mutation site detection result of a corresponding chromosome location. After all mutation detection operations are completed, mutation detection results are summarized and output to a user or another application.

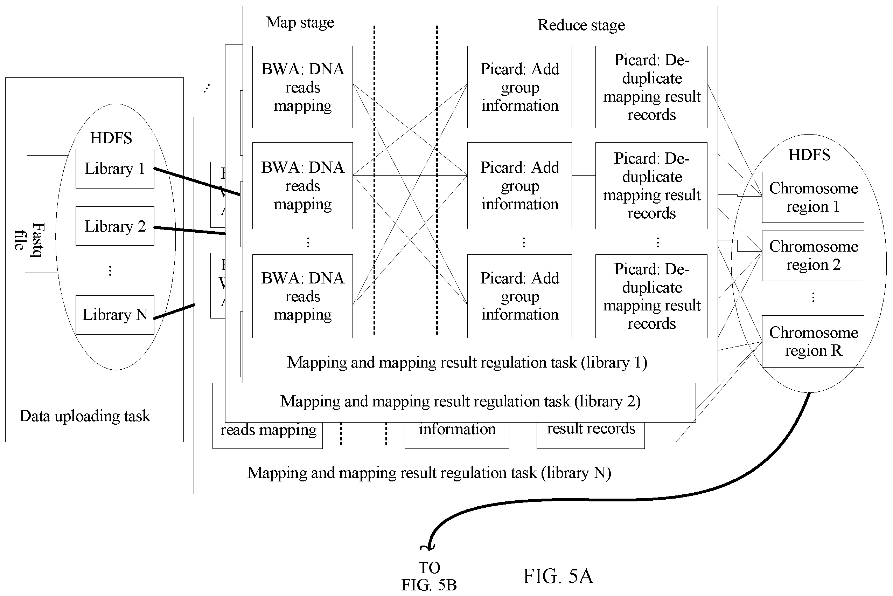

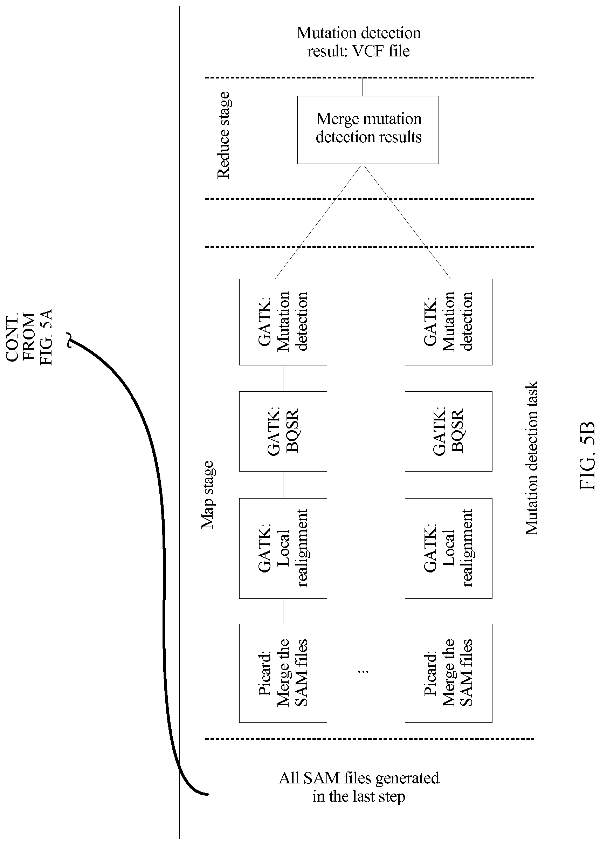

FIG. 5A and FIG. 5B are a schematic diagram in which a DNA sequence processing method runs in a Hadoop MapReduce system according to an embodiment of the present invention. For steps of the method performed by a computing device in FIG. 5A and FIG. 5B, refer to description in FIG. 4A, FIG. 4B, and FIG. 4C. Details are not described herein. FIG. 5A and FIG. 5B mainly show MapReduce tasks that need to be initiated by the computing device in a procedure of the DNA sequence processing method, and software tools that can be used in each step of the method, so as to present application of a BWA-Picard-GATK procedure in this embodiment of the present invention.

It can be learned in FIG. 5A and FIG. 5B that, the procedure of the DNA sequence processing method provided in this embodiment of the present invention is implemented in the Hadoop MapReduce system. 1+N+1 MapReduce tasks are required in total, that is, one MapReduce task is used for uploading DNA sequence data; N MapReduce tasks are used for mapping and record result regulation of N read groups, where in this process, the BWA is responsible for performing alignment computation on each data sequence read of DNA sample according to a reference sequence, and implementing sorting of result records by using a data distribution mechanism in MapReduce, and the Picard tool is responsible for steps such as de-duplicating and format conversion of the alignment result records; and the last MapReduce task is used for performing mutation detection on a gene sequence by using the GATK.

By using the method shown in FIG. 4A, FIG. 4B, and FIG. 4C or FIG. 5A and FIG. 5B, the computing device may detect a mutation status of a DNA sample according to results that are obtained after multiple sequencing libraries are used to perform sequencing on the DNA sample, so that detection precision is improved. In addition, the computing device may perform concurrently mapping and mapping record regulation operations on a read group of each sequencing library in the multiple sequencing libraries. Therefore, a time for processing the DNA sample is shortened, and mutated gene detection efficiency is improved.

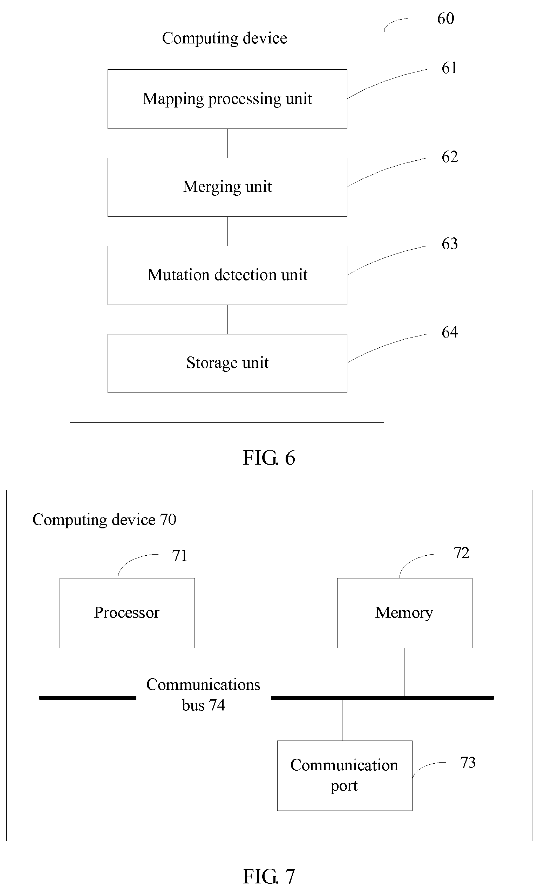

An embodiment of the present invention further provides a computing device 60. The computing device 60 is configured to implement the DNA sequence processing method provided in the foregoing method embodiment, and process N read groups of a DNA sample. Each read group includes sequence fragments reads that are obtained after a corresponding sequencing library is used to perform sequencing on the DNA sample, and N is a positive integer greater than 1. As shown in FIG. 6, the computing device 60 includes:
a mapping processing unit 61, configured to perform the following operations on each read group concurrently: performing alignment computation on each read in the read group according to a reference sequence of a chromosome, to obtain an alignment result record of the read relative to the reference sequence; determining, according to the alignment result record, a chromosome region in which each read is located, where the chromosome includes at least one chromosome region; and merging, into one intermediate result file, alignment result records of reads located in a same chromosome region, where alignment computation is performed on each read group according to a same reference sequence of a chromosome, chromosome regions included in the chromosome are the same, and after the foregoing operations are performed on each read group, each chromosome region is corresponding to N intermediate result files;
a merging unit 62, configured to determine a target sequence file of each chromosome region according to the N intermediate result files corresponding to the chromosome region; and
a mutation detection unit 63, configured to: perform mutation detection on the target sequence file of each chromosome region, to determine mutation site information of the chromosome region.

It should be noted that one sequencing library may include multiple corridors, and each corridor has a different configuration and focuses on a different aspect of DNA sequencing. The read group includes data sequences reads of all corridors in one library. The present invention sets no limitation to construction of the sequencing library.

By using the computing device 60, the computing device 60 performs an alignment computation operation on a read group of each sequencing library concurrently. Therefore, a time for processing the DNA sample is shortened, and DNA sequence processing efficiency is improved. In addition, a gene mutation status of the DNA sample is detected according to results that are obtained after multiple sequencing libraries are used to perform sequencing on the DNA sample. Therefore, in comparison with a single sequencing library, detection precision is improved in the DNA sequence processing method provided in the present invention.

Optionally, the computing device 60 further includes a storage unit 64, configured to individually save each read group in the N read groups into a distributed storage system before the mapping processing unit 61 performs the operations on the N read groups concurrently. Individual storage helps distinguish between different read groups, and helps save an intermediate file generated in a processing process.

Optionally, the storage unit 64 is specifically configured to divide each read group into at least one data block for saving. The mapping processing unit 61 is configured to perform, concurrently according to the reference sequence, alignment computation on a data block corresponding to each read. In this way, data blocks in a read group and read groups of sequencing libraries are processed concurrently, so that processing efficiency is improved.

Optionally, the alignment result record includes an identifier of a chromosome on which each read in the read group is located and location information of the read on the chromosome. The mapping processing unit 61 is configured to determine, according to the identifier of the chromosome and the location information, the chromosome region in which each read is located on the chromosome. In a possible implementation of this embodiment of the present invention, the reference sequence includes all sequences of the chromosome. A DNA sequence fragment read is aligned with the reference sequence, to determine a region that is in the reference sequence and that is most similar to the read, so as to determine location information of the read on the chromosome.

Optionally, the merging unit 62 is specifically configured to perform operations that include at least sorting and de-duplicating on all the alignment result records in the same chromosome region, to obtain the intermediate result file. Specifically, a Picard tool may be used to perform regulation operations such as sorting and de-duplicating on the alignment result records, to obtain the intermediate result file. The intermediate result file indicates a data sequence that is of a chromosome region and that is determined after DNA sequence determining is performed on a read group that is obtained by means of sequencing by using a single sequencing library.

The unit division performed on the computing device 60 is merely logical function division and may be other division in actual implementation. For example, the mapping processing unit 61 and the merging unit 62 may be integrated into one processing unit. In addition, physical implementation of the foregoing functional units may be implemented in multiple manners. The present invention sets not limitation thereto.

It may be clearly understood by persons skilled in the art that, for the purpose of convenient and brief description, for a detailed working process of the units of the described computing device, refer to a corresponding process of the foregoing method embodiments. Details are not described herein.

An embodiment of the present invention further provides another computing device 70. As shown in FIG. 7, the computing device 70 includes a processor 71, a memory 72, a communication port 73, and a communications bus 74. The processor 71, the memory 72, and the communication port 73 complete mutual communication by using the communications bus 74.

The processor 71 may be a multi-core central processing unit (Central Processing Unit, CPU), or may be an application-specific integrated circuit (Application Specific Integrated Circuit, ASIC), or may be one or more integrated circuits configured to implement this embodiment of the present invention.

The memory 72 is configured to save program code. The program code includes a computer operation instruction and a network-flow graph. The memory 72 may include a high-speed random access memory (Random Access Memory, RAM), or may further include a nonvolatile memory, for example, at least one disk memory.

The communication port 73 is configured to implement communication between the computing device 70 and another device.

The processor 71 is configured to execute the program code in the memory 72, to implement the DNA sequence processing method provided in the foregoing method embodiment. The method is used to process N read groups of a deoxyribonucleic acid DNA sample, each read group includes sequence fragments reads that are obtained after a corresponding sequencing library is used to perform sequencing on the DNA sample, N is a positive integer greater than 1, and the method includes:
performing the following operations on each read group concurrently:
performing alignment computation on each read in the read group according to a reference sequence of a chromosome, to obtain an alignment result record of the read relative to the reference sequence;
determining, according to the alignment result record, a chromosome region in which each read is located, where the chromosome includes at least one chromosome region; and
merging, into one intermediate result file, alignment result records of reads located in a same chromosome region, where
alignment computation is performed on each read group according to a same reference sequence of a chromosome, chromosome regions included in the chromosome are the same, and after the foregoing operations are performed on each read group, each chromosome region is corresponding to N intermediate result files;
determining a target sequence file of each chromosome region according to the N intermediate result files corresponding to the chromosome region; and
performing mutation detection on the target sequence file of each chromosome region, to determine mutation site information of the chromosome region.

Optionally, before the performing the operations on each read group concurrently, the method further includes:
individually saving each read group into a distributed storage system.

Optionally, the individually saving each read group into a distributed storage system includes:
dividing each read group into at least one data block for saving; and
the performing alignment computation on each read in the read group according to a reference sequence of a chromosome includes:
   performing, concurrently according to the reference sequence, alignment computation on a data block corresponding to each read.

Optionally, the alignment result record includes an identifier of a chromosome on which each read is located and location information of the read on the chromosome, and the determining, according to the alignment result record, a chromosome region in which each read is located includes:
determining, according to the identifier of the chromosome and the location information, the chromosome region in which each read is located on the chromosome.

Optionally, the merging, into one intermediate result file, alignment result records of reads located in a same chromosome region includes:
performing operations that include at least sorting and de-duplicating on the alignment result records of the reads located in the same chromosome region, to obtain the intermediate result file.

The processor 71 in this embodiment of the present invention may be a central processing unit CPU. To save computing resources of the CPU, the processor 71 may be a field programmable gate array (Field Programmable Gate Array, FPGA) or other hardware. In addition, persons skilled in the art should understand that the processor 71 may complete the operations by cooperating with another device. For ease of description, in this embodiment of the present invention, it is uniformly described as follows: The processor 71 performs the DNA sequence processing operations. For details, refer to the method steps performed by the computing device described in the method embodiment. Details are not described herein.

In the several embodiments provided in this application, it should be understood that the disclosed system, apparatus, and method may be implemented in another manner. For example, the described apparatus embodiment is merely an example. For example, the unit division is merely logical function division and may be other division in actual implementation. For example, multiple units or components may be combined or integrated into another system, or some features may be ignored or not performed. In addition, the displayed or discussed mutual couplings or direct couplings or communication connections may be implemented through some interfaces, and indirect couplings or communication connections between the apparatuses or units may be electrical connections, mechanical connections, or connections in another form.

The units described as separate parts may be or may not be physically separate, and parts displayed as units may be or may not be physical units, may be located in one position, or may be distributed on multiple network units. Some or all of the units may be selected according to actual needs to achieve the objectives of the solutions of the embodiments.

In addition, functional units in the embodiments of the present invention may be integrated into one processing unit, or each of the units may exist alone physically, or at least two units are integrated into one unit. The integrated unit may be implemented in a form of hardware, or may be implemented in a form of hardware in addition to a software functional unit.

The foregoing integrated unit implemented in a form of a software functional unit may be saved in a computer readable storage medium. The software functional unit is saved in a storage medium and includes several instructions for instructing a computer device (which may be a personal computer, a server, a network device, or the like) to perform some of the steps of the methods described in the embodiments of the present invention. The foregoing storage medium includes any medium that can save program code, such as a USB flash drive, a removable hard disk, a read-only memory (Read-Only Memory, ROM), a random access memory (Random Access Memory, RAM), a magnetic disk, or an optical disc.

## Claims

1. A computer-implemented DNA sequence processing method, **characterized in that** the method is used to process N read groups of a deoxyribonucleic acid, DNA sample, each read group comprises sequence fragments reads that are obtained after a corresponding sequencing library is used to perform sequencing on the DNA sample, N is a positive integer greater than 1, and the method comprises:
performing the following operations on each read group concurrently:
performing alignment computation on each read in the read group according to a reference sequence of a chromosome, to obtain an alignment result record of the read relative to the reference sequence;
determining, according to the alignment result record, a chromosome region in which each read is located, wherein the chromosome comprises more than one chromosome region; and
merging, into one intermediate result file, alignment result records of reads located in a same chromosome region, wherein
alignment computation is performed on each read group according to a same reference sequence of a chromosome, chromosome regions comprised in the chromosome are the same, and after the foregoing operations are performed on each read group, each chromosome region is corresponding to N intermediate result files;
determining a target sequence file of each chromosome region according to the N intermediate result files corresponding to the chromosome region, wherein the N intermediate result files corresponding to each chromosome region are merged to generate the target sequence file; and
performing mutation detection on the target sequence file of each chromosome region, to determine mutation site information of the chromosome region.

2. The method according to claim 1, wherein before the performing the operations on each read group concurrently, the method further comprises:
individually saving each read group into a distributed storage system.

3. The method according to claim 2, wherein the individually saving each read group into a distributed storage system comprises:
dividing each read group into at least one data block for saving; and
the performing alignment computation on each read in the read group according to a reference sequence of a chromosome comprises:
performing, concurrently according to the reference sequence, alignment computation on a data block corresponding to each read.

4. The method according to any one of claims 1 to 3, wherein the alignment result record comprises an identifier of a chromosome on which each read is located and location information of the read on the chromosome, and the determining, according to the alignment result record, a chromosome region in which each read is located comprises:
determining, according to the identifier of the chromosome and the location information, the chromosome region in which each read is located on the chromosome.

5. The method according to claim 4, wherein the merging, into one intermediate result file, alignment result records of reads located in a same chromosome region comprises:
performing operations that comprise at least sorting and de-duplicating on the alignment result records of the reads located in the same chromosome region, to obtain the intermediate result file.

6. A computing device, **characterized in that** the computing device is configured to process N read groups of a deoxyribonucleic acid DNA sample, each read group comprises sequence fragments reads that are obtained after a corresponding sequencing library is used to perform sequencing on the DNA sample, N is a positive integer greater than 1, and the computing device comprises:
a mapping processing unit, configured to perform the following operations on each read group concurrently:
performing alignment computation on each read in the read group according to a reference sequence of a chromosome, to obtain an alignment result record of the read relative to the reference sequence;
determining, according to the alignment result record, a chromosome region in which each read is located, wherein the chromosome comprises more than one chromosome region; and
merging, into one intermediate result file, alignment result records of reads located in a same chromosome region, wherein
alignment computation is performed on each read group according to a same reference sequence of a chromosome, chromosome regions comprised in the chromosome are the same, and after the foregoing operations are performed on each read group, each chromosome region is corresponding to N intermediate result files;
a merging unit, configured to determine a target sequence file of each chromosome region according to the N intermediate result files corresponding to the chromosome region, wherein the N intermediate result files corresponding to each chromosome region are merged to generate the target sequence file; and
a mutation detection unit, configured to: perform mutation detection on the target sequence file of each chromosome region, to determine mutation site information of the chromosome region.

7. The computing device according to claim 6, wherein the computing device further comprises a storage unit, configured to individually save each read group into a distributed storage system before the mapping processing unit performs the operations on each read group concurrently.

8. The computing device according to claim 7, wherein the storage unit is specifically configured to:
divide each read group into at least one data block for saving; and
the mapping processing unit is configured to perform, concurrently according to the reference sequence, alignment computation on a data block corresponding to each read.

9. The computing device according to any one of claims 6 to 8, wherein the alignment result record comprises an identifier of a chromosome on which each read is located and location information of the read on the chromosome, and the mapping processing unit is configured to determine, according to the identifier of the chromosome and the location information, the chromosome region in which each read is located on the chromosome.

10. The computing device according to claim 9, wherein the merging unit is specifically configured to perform operations that comprise at least sorting and de-duplicating on the alignment result records of the reads located in the same chromosome region, to obtain the intermediate result file.

11. A computer readable medium, **characterized in that** the computer readable medium is configured to save a computer program, where the computer program includes instructions that are used to perform the method according to any of claims 1 to 5.

## Patentansprüche

1. Computerimplementiertes Verfahren zur Verarbeitung von DNA-Sequenzen, **dadurch gekennzeichnet, dass** das Verfahren zur Verarbeitung von N Lesegruppen einer Desoxyribonukleinsäure-DNA-Probe verwendet wird, wobei jede Lesegruppe Sequenzfragment-Lesevorgänge umfasst, die erhalten werden, nachdem eine entsprechende Sequenzierungsbibliothek verwendet wird, um eine Sequenzierung an der DNA-Probe durchzuführen, wobei N eine positive ganze Zahl größer als 1 ist, und das Verfahren Folgendes umfasst:
gleichzeitige Durchführung der folgenden Schritte an jeder Lesegruppe:
Durchführen einer Ausrichtungsberechnung für jeden Lesevorgang in der Lesegruppe gemäß einer Referenzsequenz eines Chromosoms, um einen Ausrichtungs-Ergebnisdatensatz des Lesevorgangs relativ zur Referenzsequenz zu erhalten;
Bestimmen, gemäß dem Ausrichtungs-Ergebnisdatensatz, einer Chromosomenregion, in der sich jeder Lesevorgang befindet, wobei das Chromosom mehr als eine Chromosomenregion umfasst; und
Zusammenführen von Ausrichtungs-Ergebnisdatensätzen von Lesevorgängen, die sich in derselben Chromosomenregion befinden, in einer Zwischenergebnisdatei, wobei
die Ausrichtungsberechnung für jede Lesegruppe gemäß einer gleichen Referenzsequenz eines Chromosoms durchgeführt wird, die im Chromosom enthaltenen Chromosomenregionen sind gleich, und nachdem die vorgenannten Operationen für jede Lesegruppe durchgeführt wurden, jede Chromosomenregion N Zwischenergebnisdateien entspricht;
bestimmen einer Zielsequenzdatei jeder Chromosomenregion entsprechend den N Zwischenergebnisdateien, die der Chromosomenregion entsprechen, wobei die N Zwischenergebnisdateien, die jeder Chromosomenregion entsprechen, zusammengeführt werden, um die Zielsequenzdatei zu erzeugen; und
Durchführung der Mutationserkennung an der Zielsequenzdatei jeder Chromosomenregion, um die Mutationsstelleninformationen der Chromosomenregion zu bestimmen.

2. Verfahren nach Anspruch 1, wobei das Verfahren vor der gleichzeitigen Durchführung der Operationen an jeder Lesegruppe ferner Folgendes umfasst:
jede Lesegruppe einzeln in einem verteilten Speichersystem zu speichern.

3. Verfahren nach Anspruch 2, wobei das individuelle Speichern jeder Lesegruppe in einem verteilten Speichersystem Folgendes umfasst:
Aufteilen jeder Lesegruppe in mindestens einen Datenblock zur Speicherung; und
die Durchführung der Ausrichtungsberechnung an jedem Lesevorgang in der Lesegruppe gemäß einer Referenzsequenz eines Chromosoms Folgendes umfasst:
gleichzeitiges Durchführen einer Ausrichtungsberechnung gemäß der Referenzsequenz für einen Datenblock, der jedem Lesevorgang entspricht.

4. Verfahren nach einem der Ansprüche 1 bis 3, wobei der Datensatz des Ausrichtungsergebnisses einen Identifikator eines Chromosoms, auf dem sich jeder Lesevorgang befindet, und Informationen über den Ort des Lesevorgangs auf dem Chromosom umfasst, und die Bestimmung, gemäß dem Datensatz des Ausrichtungsergebnisses, einer Chromosomenregion, in der sich jeder Lesevorgang befindet, Folgendes umfasst:
Bestimmung der Chromosomenregion, in der sich jeder Lesevorgang auf dem Chromosom befindet, entsprechend dem Identifikator des Chromosoms und der Ortsinformation.

5. Verfahren nach Anspruch 4, wobei das Zusammenführen von Ausrichtungs-Ergebnisdatensätzen von Lesevorgängen, die sich in einer gleichen Chromosomenregion befinden, in eine Zwischenergebnisdatei Folgendes umfasst:
Durchführen von Operationen, die zumindest das Sortieren und deduplizieren der Ausrichtungs-Ergebnisdatensätze der in derselben Chromosomenregion befindlichen Lesevorgänge umfassen, um die Zwischenergebnisdatei zu erhalten.

6. Computervorrichtung, **dadurch gekennzeichnet, dass** die Computervorrichtung so konfiguriert ist, dass sie N Lesegruppen einer Desoxyribonukleinsäure-DNA-Probe verarbeitet, wobei jede Lesegruppe Sequenzfragment-Lesevorgänge umfasst, die erhalten werden, nachdem eine entsprechende Sequenzierungsbibliothek verwendet wird, um eine Sequenzierung an der DNA-Probe durchzuführen, wobei N eine positive ganze Zahl größer als 1 ist, und die Computervorrichtung Folgendes umfasst:
eine Mapping-Verarbeitungseinheit, die so konfiguriert ist, dass sie die folgenden Operationen an jeder Lesegruppe gleichzeitig durchführt:
Durchführen einer Ausrichtungsberechnung für jeden Lesevorgang in der Lesegruppe gemäß einer Referenzsequenz eines Chromosoms, um einen Ausrichtungs-Ergebnisdatensatz des Lesevorgangs relativ zur Referenzsequenz zu erhalten;
Bestimmen, gemäß dem Ausrichtungs-Ergebnisdatensatz, einer Chromosomenregion, in der sich jeder Lesevorgang befindet, wobei das Chromosom mehr als eine Chromosomenregion umfasst; und
Zusammenführen von Ausrichtungs-Ergebnisdatensätzen von Lesevorgängen, die sich in derselben Chromosomenregion befinden, in einer Zwischenergebnisdatei, wobei
die Ausrichtungsberechnung für jede Lesegruppe gemäß einer gleichen Referenzsequenz eines Chromosoms durchgeführt wird, die im Chromosom enthaltenen Chromosomenregionen gleich sind, und nachdem die vorgenannten Operationen für jede Lesegruppe durchgeführt wurden, jede Chromosomenregion N Zwischenergebnisdateien entspricht;
Bestimmen einer Zielsequenzdatei jeder Chromosomenregion entsprechend den N Zwischenergebnisdateien, die der Chromosomenregion entsprechen, wobei die N Zwischenergebnisdateien, die jeder Chromosomenregion entsprechen, zusammengeführt werden, um die Zielsequenzdatei zu erzeugen; und
eine Mutationserkennungseinheit, die konfiguriert ist, um: eine Mutationserkennung an der Zielsequenzdatei jeder Chromosomenregion durchzuführen, um Mutationsstelleninformationen der Chromosomenregion zu bestimmen.

7. Computervorrichtung nach Anspruch 6, wobei die Computervorrichtung ferner eine Speichereinheit umfasst, die so konfiguriert ist, dass sie jede Lesegruppe einzeln in einem verteilten Speichersystem speichert, bevor die Mapping-Verarbeitungseinheit die Operationen an jeder Lesegruppe gleichzeitig ausführt.

8. Computervorrichtung nach Anspruch 7, wobei die Speichereinheit speziell zu Folgendem konfiguriert ist:
Aufteilen jeder Lesegruppe in mindestens einen Datenblock zur Speicherung; und
die Mapping-Verarbeitungseinheit so konfiguriert ist, dass sie gleichzeitig gemäß der Referenzsequenz eine Ausrichtungsberechnung an einem Datenblock durchführt, der jedem Lesevorgang entspricht.

9. Computervorrichtung nach einem der Ansprüche 6 bis 8, wobei der Datensatz des Ausrichtungsergebnisses einen Identifikator eines Chromosoms, auf dem sich jeder Lesevorgang befindet, und Informationen über den Ort des Lesevorgangs auf dem Chromosom umfasst, und die Mapping-Verarbeitungseinheit so konfiguriert ist, dass sie gemäß dem Identifikator des Chromosoms und den Ortsinformationen die Chromosomenregion bestimmt, in dem sich jeder Lesevorgang auf dem Chromosom befindet.

10. Computervorrichtung nach Anspruch 9, wobei die Mapping-Verarbeitungseinheit speziell konfiguriert ist, um Operationen durchzuführen, die zumindest das Sortieren und Deduplizieren der Ausrichtungs-Ergebnisdatensätze der in derselben Chromosomenregion befindlichen Lesevorgänge umfassen, um die Zwischenergebnisdatei zu erhalten.

11. Computerlesbares Medium, **dadurch gekennzeichnet, dass** das computerlesbare Medium zum Speichern eines Computerprogramms konfiguriert ist, wobei das Computerprogramm Anweisungen beinhaltet, die zum Ausführen des Verfahrens gemäß einem der Ansprüche 1 bis 5 verwendet werden.

## Revendications

1. Procédé de traitement de séquence d'ADN mis en œuvre par ordinateur, caractérisé en que le procédé est utilisé pour traiter N groupes de lecture d'un échantillon d'acide désoxyribonucléique, ADN, chaque groupe de lecture comprend des lectures de fragments de séquence qui sont obtenus après qu'une bibliothèque de séquençage correspondante a été utilisée pour réaliser un séquençage sur l'échantillon d'ADN, N est un entier positif supérieur à 1, et le procédé comprend :
la réalisation des opérations suivantes sur chaque groupe de lecture simultanément :
la réalisation d'un calcul d'alignement sur chaque lecture dans le groupe de lecture selon une séquence de référence d'un chromosome, pour obtenir un enregistrement de résultat d'alignement de la lecture par rapport à la séquence de référence ;
la détermination, selon l'enregistrement de résultat d'alignement, d'une région chromosomique dans laquelle chaque lecture est située, dans lequel le chromosome comprend plus d'une région chromosomique ; et
la fusion, dans un fichier de résultats intermédiaires, d'enregistrements de résultats d'alignement de lectures situées dans une même région chromosomique, dans lequel
le calcul d'alignement est réalisé sur chaque groupe de lecture selon une même séquence de référence d'un chromosome, les régions chromosomiques comprises dans le chromosome sont les mêmes, et après que les opérations précédentes ont été réalisées sur chaque groupe de lecture, chaque région chromosomique correspond à N fichiers de résultats intermédiaires ;
la détermination d'un fichier de séquence cible de chaque région chromosomique selon les N fichiers de résultats intermédiaires correspondant à la région chromosomique, dans lequel les N fichiers de résultats intermédiaires correspondant à chaque région chromosomique sont fusionnés pour générer le fichier de séquence cible ; et
la réalisation d'une détection de mutation sur le fichier de séquence cible de chaque région chromosomique, pour déterminer les informations sur le site de mutation de la région chromosomique.

2. Procédé selon la revendication 1, dans lequel, avant la réalisation simultanée des opérations sur chaque groupe de lecture, le procédé comprend également :
la sauvegarde individuelle de chaque groupe de lecture dans un système de stockage distribué.

3. Procédé selon la revendication 2, dans lequel la sauvegarde individuelle de chaque groupe de lecture dans un système de stockage distribué comprend :
la division de chaque groupe de lecture en au moins un bloc de données à des fins de sauvegarde ; et
la réalisation d'un calcul d'alignement sur chaque lecture du groupe de lecture selon une séquence de référence d'un chromosome comprend :
la réalisation, simultanément selon la séquence de référence, d'un calcul d'alignement sur un bloc de données correspondant à chaque lecture.

4. Procédé selon l'une quelconque des revendications 1 à 3, dans lequel l'enregistrement de résultat d'alignement comprend un identifiant du chromosome sur lequel chaque lecture est située et des informations d'emplacement de la lecture sur le chromosome, et la détermination, selon l'enregistrement de résultat d'alignement, d'une région chromosomique dans laquelle chaque lecture est située comprend :
la détermination, selon l'identifiant du chromosome et les informations d'emplacement, de la région chromosomique dans laquelle chaque lecture est située sur le chromosome.

5. Procédé selon la revendication 4, dans lequel la fusion, en un seul fichier de résultats intermédiaires, d'enregistrements de résultats d'alignement de lectures situées dans une même région chromosomique comprend :
la réalisation d'opérations qui comprennent au moins le tri et la déduplication sur les enregistrements de résultats d'alignement des lectures situées dans la même région chromosomique, pour obtenir le fichier de résultats intermédiaires.

6. Dispositif informatique, **caractérisé en ce que** le dispositif informatique est configuré pour traiter N groupes de lecture d'un échantillon d'acide désoxyribonucléique, ADN, chaque groupe de lecture comprend des lectures de fragments de séquence qui sont obtenus après qu'une bibliothèque de séquençage correspondante a été utilisée pour réaliser un séquençage sur l'échantillon d'ADN, N est un entier positif supérieur à 1, et le dispositif informatique comprenant :
une unité de traitement de mappage, configurée pour réaliser simultanément les opérations suivantes sur chaque groupe de lecture :
la réalisation d'un calcul d'alignement sur chaque lecture dans le groupe de lecture selon une séquence de référence d'un chromosome, pour obtenir un enregistrement de résultat d'alignement de la lecture par rapport à la séquence de référence ;
la détermination, selon l'enregistrement de résultat d'alignement, d'une région chromosomique dans laquelle chaque lecture est située, dans lequel le chromosome comprend plus d'une région chromosomique ; et
la fusion, dans un fichier de résultats intermédiaires, d'enregistrements de résultats d'alignement de lectures situées dans une même région chromosomique, dans lequel
le calcul d'alignement est réalisé sur chaque groupe de lecture selon une même séquence de référence d'un chromosome, les régions chromosomiques comprises dans le chromosome sont les mêmes, et après que les opérations précédentes ont été réalisées sur chaque groupe de lecture, chaque région chromosomique correspond à N fichiers de résultats intermédiaires ;
une unité de fusion, configurée pour déterminer un fichier de séquence cible de chaque région chromosomique selon les N fichiers de résultats intermédiaires correspondant à la région chromosomique, dans lequel les N fichiers de résultats intermédiaires correspondant à chaque région chromosomique sont fusionnés pour générer le fichier de séquence cible ; et
une unité de détection de mutation, configurée pour : réaliser une détection de mutation sur le fichier de séquence cible de chaque région chromosomique, pour déterminer les informations sur le site de mutation de la région chromosomique.

7. Dispositif informatique selon la revendication 6, dans lequel le dispositif informatique comprend également une unité de stockage, configurée pour sauvegarder individuellement chaque groupe de lecture dans un système de stockage distribué avant que l'unité de traitement de mappage ne réalise les opérations sur chaque groupe de lecture simultanément.

8. Dispositif informatique selon la revendication 7, dans lequel l'unité de stockage est spécifiquement configurée pour :
diviser chaque groupe de lecture en au moins un bloc de données à des fins de sauvegarde ; et
l'unité de traitement de mappage est configurée pour réaliser, simultanément selon la séquence de référence, un calcul d'alignement sur un bloc de données correspondant à chaque lecture.

9. Dispositif informatique selon l'une quelconque des revendications 6 à 8, dans lequel l'enregistrement de résultat d'alignement comprend un identifiant d'un chromosome sur lequel chaque lecture est située et des informations d'emplacement de la lecture sur le chromosome, et l'unité de traitement de mappage est configurée pour déterminer, selon l'identifiant du chromosome et les informations d'emplacement, la région chromosomique dans laquelle chaque lecture est située sur le chromosome.

10. Dispositif informatique selon la revendication 9, dans lequel l'unité de fusion est spécifiquement configurée pour réaliser des opérations qui comprennent au moins le tri et la déduplication sur les enregistrements de résultats d'alignement des lectures situées dans la même région chromosomique, pour obtenir le fichier de résultats intermédiaires.

11. Support lisible par ordinateur, **caractérisé en ce que** le support lisible par ordinateur est configuré pour sauvegarder un programme informatique, où le programme informatique comporte des instructions qui sont utilisées pour réaliser le procédé selon l'une quelconque des revendications 1 à 5.
